# EUROPEAN PATENT APPLICATION

(11) **EP 4 245 348 A1**
(43) Date of publication of application: **20.09.2023**
(21) Application number: 22305297.8
(22) Date of filing: 15.03.2022
(51) Int. Cl.: A61M 21/02

(54) **DIGITAL THERAPEUTIC COMPOSITION AND USES THEREOF IN THERAPY**

(71) Applicant: Lucine, 33300 Bordeaux (FR)
(72) Inventor: The designation of the inventor has not yet been filed
(74) Representative: Icosa

(57) **Abstract**

The present invention relates to a digital therapeutic composition, comprising:
- at least one oratorical soundtrack;
- at least one sequence of alpha binaural beats; and
- at least one sequence of theta binaural beats;
and further optionally comprising:
- an immersive video stream simulating a virtual reality environment;
- at least one soundtrack comprising sounds of nature; and/or
- at least one sequence of alternating bilateral stimulations.

It also relates to a therapeutic use for treating pain, and to a non-therapeutic method of relaxation.

## Description

### FIELD OF INVENTION

The present invention relates to the field of digital therapeutics (DTx) and digital therapeutic systems.

### BACKGROUND OF INVENTION

Chronic pain is a pathological condition defined as a persistent or recurrent pain, lasting for at least three months. It can lead to critical dysfunctions in both peripherical and central nervous systems, including grey and white matter loss, increase or decrease of the activity in major cerebral areas, alterations of synaptic neurotransmission, etc. In addition, chronic pain can severely affect the lifestyle of patients suffering from it: from nutrition and physical activity, to sleep disorders and mental wellbeing. Chronic pain can appear through aging, but also emerges in patients presenting specific diseases, such as in women diagnosed with endometriosis.

Endometriosis is a pathology that has seen its study exponentially increased in the last century. It is characterized by a lesioned endometrial tissue migrating outside the uterus, mainly on the pelvic peritoneum, ovaries, and rectovaginal septum. In rarer cases, it might also reach deeper tissues, such as the diaphragm pleura and the pericardium. Endometriosis is a multifactorial disease, resulting from the combined action of genetic and environmental factors. In this manner, abnormalities of the immune system or the angiogenesis, as well as biochemical factors, play a crucial role in specific biological pathways promoting the growth of endometrial lesions. The lesions of the endometrial tissue and their migration are major contributors to chronic pelvic pain and infertility among women.

The mean prevalence of endometriosis in women with chronic pelvic pain has been estimated at 70 ± 3 %, but might varies from 2 % to 93 % depending on the studies. Similarly, this percentage in women with acute pelvic pain is reaching over 33 %. An annual incidence of about 1/1000 has been reported in women aged 15-49. Among the numerous painful symptoms of endometriosis, the most common ones are dysmenorrhea and profound dyspareunia, appearing in nearly 80 % and 30 % of the patients, respectively. Dyschesia/dysuria or intermenstrual pelvic pain are less frequently reported, and usually associated with rectal and bladder lesions or ovulation, respectively. All these pains can be nociceptive, neuropathic, or nociplastic (a combination of the previous ones); endometriosis frequently generating all three types of pain.

Current management of endometriosis pain involves pharmacological and/or surgical treatments. The goals of these medical therapies are, for instance, to inhibit ovulation, suppress menstruation, or achieve a stable steroidal hormonal environment. These are based on the concept that the response of the eutopic and ectopic endometrium is substantially similar.

Nevertheless, besides not being adapted for women willing to get pregnant, the pharmacological approaches are not always sufficient to reduce endometriosis pain. Thus, patients who are ineligible, refractory, or not fully responsive to drug treatments, as well as those ineligible for surgical treatments, lack of pain-reliever solutions. Moreover, recurrence of pain after surgery, with or without additional hormonal therapy, is common.

There is thus a need for the development of non-pharmacological and non-surgical alternatives for managing and alleviating pain, in particular endometriosis pain.

Digital therapeutics (DTx) are an evolving field, defined as treatments or therapies that utilize digital products rather than pharmacological products to improve health outcomes. DTx have been or are being developed for treating or managing a variety of conditions, including type II diabetes, Alzheimer's disease, dementia, congestive heart failure, chronic obstructive pulmonary disease, asthma, lung disease, obesity, substance abuse, attention deficit hyperactivity disorder, insomnia, hypertension, anxiety, depression, etc.

Here, the Inventors have developed a digital therapeutic solution that proved efficient at alleviating pain, in particular chronic pelvic pain.

### SUMMARY

The invention relates to a digital therapeutic composition, comprising:
- at least one oratorical soundtrack;
- at least one sequence of alpha binaural beats; and
- at least one sequence of theta binaural beats;
and further optionally comprising:
- an immersive video stream simulating or generating a virtual reality environment;
- at least one soundtrack comprising sounds of nature; and/or
- at least one sequence of alternating bilateral stimulations.

In some embodiments, the oratorical soundtrack comprises a hypnosis script, preferably said hypnosis script is spoken by a woman characterized by a voice frequency ranging from about 150 to about 300 Hz.

In some embodiments, the oratorical soundtrack comprises:
- a first sequence of hypnosis induction;
- a second sequence of hypnosis injunction; and
- a third sequence of hypnosis exit.

In some embodiments, the at least one sequence of alpha binaural beats and the at least one sequence of theta binaural beats each independently comprise two dichotic soundtracks, wherein each dichotic soundtrack comprises a pure-tone sine wave with a frequency ranging from about 450 to about 500 Hz.

In some embodiments, the virtual reality environment comprises at least one sequence of images in natural environment or in a fantasy environment.

In some embodiments, the sounds of nature are linked or related to at least one sequence of images of the virtual reality environment.

In some embodiments, the at least one sequence of alternating bilateral stimulations comprises a visual stimulus.

In some embodiments, the visual stimulus moves in a rhythmic left-right pattern with a frequency ranging from about 0.2 Hz to about 2 Hz, preferably with a frequency of about 0.8 Hz.

In some embodiments, the composition comprises:
- an oratorical soundtrack,
- one sequence of alpha binaural beats,
- one sequence of theta binaural beats,
- an immersive video stream simulating a virtual reality environment,
- a soundtrack comprising sounds of nature, and
- at least one sequence of alternating bilateral visual stimulations.

The invention also relates to the digital therapeutic composition according to the invention, for use in therapy.

The invention also relates to the digital therapeutic composition according to the invention, for use in the treatment of pain in a subject in need thereof.

In some embodiments, pain is pelvic pain, preferably chronic pelvic pain, more preferably endometriosis-related pain.

In some embodiments, the virtual reality environment and the at least one oratorical soundtrack are to be administered to the subject in need thereof concomitantly and for the entire duration of the care.

In some embodiments, the at least one sequence of alpha binaural beats, the at least one sequence of theta binaural beats and the at least one sequence of alternating bilateral stimulations are to be administered to the subject in need thereof in the following order:
- the at least one sequence of alpha binaural beats is to be administered astride a first sequence of hypnosis induction and a second sequence of hypnosis injunction; then
- a first sequence of alternating bilateral stimulations is to be administered during the second sequence of hypnosis injunction; then
- the at least one sequence of theta binaural beats is to be administered during the second sequence of hypnosis injunction; then
- optionally, a second sequence of alternating bilateral stimulations is to be administered during the second sequence of hypnosis injunction.

The invention also relates to a non-therapeutic method of relaxation, comprising subjecting a subject to the digital therapeutic composition according to the invention.

### DEFINITIONS

In the present invention, the following terms have the following meanings.

"**Oratorical soundtrack**" refers to a soundtrack comprising a spoken speech.

"**Hypnosis script**" refers to a speech comprising hypnotic language which, when recited to a subject, induces a hypnotic state or a state of trance of said subject. Hypnosis scripts are typically divided into several parts, including (i) hypnosis induction (*i.e.*, pre-hypnotic instructions or suggestions given to the subject to enter a hypnotic state), (ii) hypnosis injunctions (*i.e.*, hypnotic instructions or suggestions given to the subject to elicit specific outcomes), and (iii) hypnosis exit (*i.e.*, post-hypnotic instructions or suggestions given to the subject to exit the hypnotic state).

"**Binaural beat**" refers to an auditory illusion perceived when two different pure-tone sine waves, both with frequencies typically lower than 1500 Hz, and with 1 Hz to 300 Hz or more difference between them, are dichotically presented to a subject (*i.e.*, one through each ear). For illustration purposes, a pure tone of 450 Hz can be presented in one ear of the subject and the same pure tone but shifted to 460 Hz can be presented to the other ear of the subject. The subject will not perceive the two tones separately; instead, a single tone will be perceived, whose lateralization moves across the head at a rate of 10 Hz, from centered, to one side, then from the other side back to the center, and so on. Binaural beats are categorized as delta, theta, alpha, beta and gamma, depending on the difference of sine wave frequency presented in each ear of the subject.

"**Alpha binaural beat**" refers to binaural beats characterized by a dichotic difference of sine wave frequency presented in each ear of the subject ranging from about 9 Hz to about 14 Hz.

"**Theta binaural beat**" refers to binaural beats characterized by a dichotic difference of sine wave frequency presented in each ear of the subject ranging from about 4 Hz to about 8 Hz.

"**Virtual reality environment**" refers to an immersive, computer-generated environment. Common systems for generating a virtual reality environment include head mounted displays such as virtual reality headsets, and multi-projected environments, both of which generate an immersive video stream that simulates a subject's physical presence in a virtual environment. It is typically possible to explore a virtual reality environment to some extent, *e*.*g*., by remaining at a given position in the environment and looking around, or by changing position in the environment.

"**Immersive video stream**" refers to a 360°-degree video, which comprises at least one sequence of images of a same scene or of a same environment for multiple directions and, optionally, from multiple positions, at a same time. It might be based on live-captured images of a real scene or based on computer-generated images.

"**Head-mounted display**" refers to a virtual reality headset or to any suitable device intended to be worn by a user's head and covering the user's eyes, the device being provided with an electronic display, one or more position and/or motion sensors and a controlling unit. The electronic display might comprise a single electronic display or multiple electronic displays (*e*.*g*., a display for each eye of a user). The device might be designed to limit the peripheral vision of the user with using blinders on the sides and top of the device. The controlling unit might be embedded into the device or it might reside on separate hardware, such as a dedicated computer, a smartphone, or a distant server.

"**Storage medium**" is intended to include random access memory (RAM) as dynamic random-access memory (DRAM) or static random-access memory (SRAM), read-only memory (ROM), and/or other types of memory. It might be embedded into the head mounted display or it might reside on separate hardware, such as a dedicated computer, a smartphone, or a distant server.

"**Alternating bilateral stimulation**" refers to stimuli applied to a subject and which occur in a rhythmic left-right pattern. Alternating bilateral stimulations can consist of visual, auditory or tactile stimuli.

"**Pelvic pain**" refers to a pain in the lowest part of the abdomen and in the pelvis. Pelvic pain may be a symptom arising from the reproductive, urinary or digestive systems, or from muscles and ligaments in the pelvis. Pelvic pain can be dull or sharp, can be constant or intermittent, and can be mild, moderate or severe. Pelvic pain can occur briefly (*i.e.*, acute pelvic pain) or over the long term (*i.e.*, chronic pelvic pain). In particular, chronic pelvic pain typically refers to any constant or intermittent pelvic pain that has been present for six months or more.

"**Treatment**" and any declension thereof refers to both therapeutic treatment and prophylactic or preventative measures. Typically, the aim of a treatment is to prevent, slow down, alleviate, or partially or totally cure a targeted pathologic condition or disorder (*e*.*g*., pain, in particular, pelvic pain). Those in need of treatment include those already affected with the pathologic condition or disorder, as well as those prone to develop the pathologic condition or disorder and those in whom the pathologic condition or disorder is to be prevented.

### DETAILED DESCRIPTION

This invention relates to a digital therapeutic composition, comprising:
- at least one oratorical soundtrack;
- at least one sequence of alpha binaural beats; and
- at least one sequence of theta binaural beats.

The digital therapeutic composition comprises at least one oratorical soundtrack, which comprises or consists of a spoken hypnosis script.

In some embodiments, the spoken hypnosis script comprises:
a) a first sequence of hypnosis induction;
b) a second sequence of hypnosis injunction; and
c) a third sequence of hypnosis exit.

In some embodiments, the first sequence of hypnosis induction lasts for a period of time ranging from about 90 seconds to about 180 seconds, such as for 90, 100, 110, 120, 130, 140, 150, 160, 170 or 180 seconds. In some embodiments, the first sequence of hypnosis induction lasts for a period of time of 130 seconds.

In some embodiments, the second sequence of hypnosis injunction lasts for a period of time ranging from about 600 seconds to about 1200 seconds, such as for 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190 or 1200 seconds. In some embodiments, the second sequence of hypnosis injunction lasts for a period of time of 950 seconds.

In some embodiments, the third sequence of hypnosis exit lasts for a period of time ranging from about 120 seconds to about 240 seconds, such as for 120, 130, 140, 150, 160, 170, 180, 190, 200, 210, 220, 230 or 240 seconds. In some embodiments, the third sequence of hypnosis exit lasts for a period of time of 180 seconds.

In some embodiments, the spoken hypnosis script comprises:
a) a first sequence of hypnosis induction lasting for about 90 to about 180 seconds, preferably lasting for about 130 seconds;
b) a second sequence of hypnosis injunction lasting for about 600 to about 1200 seconds, preferably lasting for about 950 seconds; and
c) a third sequence of hypnosis exit lasting for about 120 to about 240 seconds, preferably lasting for about 180 seconds.

In some embodiments, the first sequence of hypnosis induction, the second sequence of hypnosis injunction and the third sequence of hypnosis exit each comprise subsequences of predefined sentences spoken by a human voice.

In some embodiments, the spoken hypnosis script is divided into subsequences, including several or all, either unique or multiple, of the following: a spoken welcoming subsequence, a spoken induction subsequence, a spoken dissociation subsequence, a spoken questioning subsequence (*e*.*g*., about bodily sensations), a spoken sensory openness subsequence, a spoken post-hypnotic suggestion subsequence.

In some embodiments, the spoken hypnosis script is spoken by a woman. Typically, a woman's voice frequency ranges from about 150 Hz to about 300 Hz.

In some embodiments, the spoken hypnosis script is spoken by a woman whose voice rhythm, voice pitch, voice loudness and/or voice harmonic-to-noise ratio, and optionally voice frequency, decreases along the second sequence of hypnosis injunction.

The digital therapeutic composition comprises at least one sequence of alpha binaural beats, which are characterized by a dichotic frequency difference ranging from about 9 to about 14 Hz.

In some embodiment, the alpha binaural beats are characterized by a dichotic frequency difference of about 10 Hz

In some embodiment, the at least one sequence of alpha binaural beats comprises or consists of two dichotic soundtracks, each comprising or consisting of a pure-tone sine wave with a frequency ranging from about 450 to about 500 Hz.

In some embodiments, the at least one sequence of alpha binaural beats comprises or consists of two dichotic soundtracks, each comprising or consisting of a pure-tone sine wave, each pure-tone sine wave independently from each other having a frequency of about 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499 or 500 Hz.

In some embodiments, the at least one sequence of alpha binaural beats comprises or consists of two dichotic soundtracks, each comprising or consisting of a pure-tone sine wave, wherein a first pure-tone sine wave is presented in the right ear of a subject and has a frequency lower than a second pure-tone sine wave presented in the left ear of the subj ect.

In some embodiments, the at least one sequence of alpha binaural beats comprises or consists of two dichotic soundtracks, each comprising or consisting of a pure-tone sine wave, wherein a first pure-tone sine wave has a frequency of about 456 Hz, and a second pure-tone sine wave has a frequency of about 466 Hz, preferably the first pure-tone sine wave is presented in the right ear of a subject and the second pure-tone sine wave is presented in the left ear of the subject.

In some embodiments, the at least one sequence of alpha binaural beats lasts of a period of time ranging from about 120 to about 360 seconds, such as for 120, 130, 140, 150, 160, 170, 180, 190, 200, 210, 220, 230, 240, 250, 260, 270, 280, 290, 300, 310, 320, 330, 340, 350 or 360 seconds. In some embodiments, the at least one sequence of alpha binaural beats lasts for a period of time of 240 seconds

The digital therapeutic composition comprises at least one sequence of theta binaural beats, which are characterized by a dichotic frequency difference ranging from about 4 to about 8 Hz.

In some embodiment, the theta binaural beats are characterized by a dichotic frequency difference of about 6 Hz

In some embodiment, the at least one sequence of theta binaural beats comprises or consists of two dichotic soundtracks, each comprising or consisting of a pure-tone sine wave with a frequency ranging from about 450 to about 500 Hz.

In some embodiments, the at least one sequence of theta binaural beats comprises or consists of two dichotic soundtracks, each comprising or consisting of a pure-tone sine wave, each pure-tone sine wave independently from each other having a frequency of about 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499 or 500 Hz.

In some embodiments, the at least one sequence of theta binaural beats comprises or consists of two dichotic soundtracks, each comprising or consisting of a pure-tone sine wave, wherein a first pure-tone sine wave is presented in the right ear of a subject and has a frequency lower than a second pure-tone sine wave presented in the left ear of the subj ect.

In some embodiments, the at least one sequence of theta binaural beats comprises or consists of two dichotic soundtracks, each comprising or consisting of a pure-tone sine wave, wherein a first pure-tone sine wave has a frequency of about 460 Hz, and a second pure-tone sine wave has a frequency of about 466 Hz, preferably the first pure-tone sine wave is presented in the right ear of a subject and the second pure-tone sine wave is presented in the left ear of the subject.

In some embodiments, the at least one sequence of theta binaural beats lasts of a period of time ranging from about 120 to about 360 seconds, such as for 120, 130, 140, 150, 160, 170, 180, 190, 200, 210, 220, 230, 240, 250, 260, 270, 280, 290, 300, 310, 320, 330, 340, 350 or 360 seconds. In some embodiments, the at least one sequence of theta binaural beats lasts for a period of time of 240 seconds.

In some embodiments, the digital therapeutic composition further comprises an immersive video stream.

In some embodiment, the immersive video stream simulates or generates a virtual reality environment.

In some embodiments, the virtual reality environment comprises or consists of at least one sequence of images displaying or representing a natural environment.

Natural environments include, but are not limited to, bodies of waters and their shores, such as, *e.g.*, oceans, seas, rivers, lakes, ponds, lagoons, coves, fjords, bays, and the like; meadows, fields, grasslands and the like; forests; hills, mountains; and combinations thereof.

In some embodiments, the virtual reality environment comprises or consists of at least one sequence of images displaying or representing an imaginary or fantasy environment.

By "imaginary or fantasy environment", it is meant an environment which is not natural, *i.e.*, unreal.

In some embodiments, the immersive video stream simulates or generates a virtual reality environment and further comprises at least one subset of images representing a graphical trigger element that appears, moves and/or disappears in said virtual reality environment.

It is desirable in particular that graphical trigger elements appear, move and/or disappear in the virtual reality environment to avoid any monotony and boredom of a subject immersed in the virtual reality environment. Examples of such graphical trigger elements include, but are not limited to, a shooting star sailing through the sky, a cloud of fireflies flying away, bursting bubbles, an apparition of a rainbow, a flower that opens, and the like.

In some embodiments, the immersive video stream comprises a first sequence of images simulating or generating the virtual reality environment with a first dominant color, immediately followed by a second sequence of images simulating or generating the virtual reality environment in natural (or realistic) colors, immediately followed by a third sequence of images simulating or generating the virtual reality environment with a third dominant color preferably distinct from the first dominant color.

In some embodiments, the first dominant color is a warm color, *e*.*g*., yellow, red or any shades thereof. In some embodiments, the third dominant color is a cold color, *e.g.*, blue or any shades thereof.

In some embodiments, the digital therapeutic composition further comprises at least one soundtrack comprising sounds of nature.

Examples of sounds of nature include, but are not limited to, water sounds (*e*.*g*., ocean waves, flowing river, rainfall, waterfall, gentle stream, etc.), wind sounds, animal sounds (*e*.*g*., bird songs, etc.), and combinations thereof.

In some embodiments, the sounds of nature are linked or related to at least one sequence of images of a virtual reality environment described above.

By way of example, when the sequence of images displays or represents an ocean's shore, the soundtrack comprises sounds of ocean waves, sea wind, shore animals, etc. When the sequence of images displays or represents a forest, the soundtrack comprises sounds of wind rustling in trees, forest animals, twig cracking, etc.

In some embodiments, the digital therapeutic composition further comprises at least one sequence of alternating bilateral stimulations.

In some embodiments, the alternating bilateral stimulations are selected from the group consisting of alternating bilateral visual stimulations, alternating bilateral auditory stimulations, alternating bilateral tactile stimulations, and alternating bilateral electrical stimulations. In some embodiments, the alternating bilateral stimulations are alternating bilateral visual stimulations.

In some embodiments, the alternating bilateral visual stimulations comprise or consist of a visual stimulus moving in a rhythmic left-right pattern, *i.e.,* it moves, swings or oscillates from left to right to left, and so on.

The visual stimulus can be a shape (in 2 dimensions) or an object (in 3 dimensions). It can be regular or irregular. Non-limiting examples include a dot, a circle, a sphere, a cylinder, a square, a cube, a cuboid, a triangle, a pyramid, a cone, a polygon, etc.

In some embodiment where the composition comprises a virtual reality environment, the visual stimulus may be an element of the virtual reality environment, or alternatively can be superimposed on top of the images displaying or representing the virtual reality environment.

In some embodiments where the composition comprises an immersive video stream simulating or generating a virtual reality environment, graphical trigger elements do not appear, move and/or disappear in said virtual reality environment concomitantly with alternating bilateral visual stimulations.

In some embodiment, the visual stimulus is of uniform color.

In some embodiment, the visual stimulus if of a color selected from the group consisting of white, silver, gray, yellow, brown, blue, and any shades thereof.

In some embodiment, the visual stimulus is not red, black, or any shades thereof.

In some embodiments, the visual stimulus moves in a rhythmic left-right pattern with a frequency ranging from about 0.2 Hz to about 2 Hz, such as about 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 Hz, *i.e.,* the time necessary for the visual stimulus to move from centered, to one side, then from the other side back to the center, ranges from about to 0.5 seconds about 5 seconds.

. In some embodiments, the visual stimulus moves in a rhythmic left-right pattern with a frequency of about 0.8 Hz, *i.e.*, the time necessary for the visual stimulus to move from centered, to one side, then from the other side back to the center, is of about 1.25 seconds.

In some embodiments, the at least one sequence of alternating bilateral visual stimulations lasts of a period of time ranging from about 120 seconds to about 240 seconds, such as for 120, 130, 140, 150, 160, 170, 180, 190, 200, 210, 220, 230 or 240 seconds. In some embodiments, the at least one sequence of alternating bilateral visual stimulations lasts for a period of time of 180 seconds.

In some embodiments, the at least one sequence of alternating bilateral visual stimulations lasts of a period of time ranging from about 240 seconds to about 480 seconds, such as for 240, 250, 260, 270, 280, 290, 300, 310, 320, 330, 340, 350, 360, 370, 380, 390, 400, 410, 420, 430, 440, 450, 460, 470 or 480 seconds. In some embodiments, the at least one sequence of alternating bilateral visual stimulations lasts for a period of time of 360 seconds.

In some embodiments, the at least one sequence of alternating bilateral visual stimulations comprises or consists of 2, 3, 4, 5, 6, 7 or more subsequences interrupted by rest periods, each subsequence comprising or consisting of from 10 to 20 left-right moves of the visual stimulus, such as 10, 11, 12, 13, 14, 15, 16, 17, 18, 19 or 20 left-right moves of the visual stimulus, preferably of 14 left-right moves of the visual stimulus.

By "rest period", it is meant that the visual stimulus does not move in a rhythmic left-right pattern and remains still. Alternatively, the visual stimulus can disappear during the rest period and reappear at the beginning of the following subsequence.

In some embodiment, the digital therapeutic composition comprises a single sequence of alternating bilateral visual stimulations. Preferably, the single sequence of alternating bilateral visual stimulations lasts of a period of time ranging from about 240 seconds to about 480 seconds, such as for 240, 250, 260, 270, 280, 290, 300, 310, 320, 330, 340, 350, 360, 370, 380, 390, 400, 410, 420, 430, 440, 450, 460, 470 or 480 seconds, preferably for 360 seconds. Preferably, the single sequence of alternating bilateral visual stimulations comprises or consists of 5, 6, 7 or more subsequences interrupted by rest periods, preferably of 6 subsequences interrupted by rest periods, each subsequence comprising or consisting of from 10 to 20 left-right moves of the visual stimulus, such as 10, 11, 12, 13, 14, 15, 16, 17, 18, 19 or 20 left-right moves of the visual stimulus, preferably of 14 left-right moves of the visual stimulus.

In some embodiment, the digital therapeutic composition comprises two sequences of alternating bilateral visual stimulations. Preferably, the two sequences of alternating bilateral visual stimulations each independently from each other last of a period of time ranging from about 120 seconds to about 240 seconds, such as for 120, 130, 140, 150, 160, 170, 180, 190, 200, 210, 220, 230 or 240 seconds, preferably for 180 seconds. Preferably, the two sequences of alternating bilateral visual stimulations comprise or consist of 2, 3, 4 or more subsequences interrupted by rest periods, preferably of 3 subsequences interrupted by rest periods, each subsequence comprising or consisting of from 10 to 20 left-right moves of the visual stimulus, such as 10, 11, 12, 13, 14, 15, 16, 17, 18, 19 or 20 left-right moves of the visual stimulus, preferably of 14 left-right moves of the visual stimulus.

In some embodiments, the digital therapeutic composition comprises or consists of:
- at least one oratorical soundtrack, as defined above;
- at least one sequence of alpha binaural beats, as defined above; and
- at least one sequence of theta binaural beats, as defined above;
- an immersive video stream simulating or generating a virtual reality environment, as defined above;
- at least one soundtrack comprising sounds of nature, as defined above; and
- at least one sequence of alternating bilateral visual stimulations, as defined above.

In some embodiments, the digital therapeutic composition comprises or consists of:
- one oratorical soundtrack, as defined above;
- one sequence of alpha binaural beats, as defined above; and
- one sequence of theta binaural beats, as defined above;
- an immersive video stream simulating or generating a virtual reality environment, as defined above;
- one soundtrack comprising sounds of nature, as defined above; and
- a single sequence of alternating bilateral visual stimulations, as defined above.

In some embodiments, the digital therapeutic composition comprises or consists of:
- one oratorical soundtrack, as defined above;
- one sequence of alpha binaural beats, as defined above; and
- one sequence of theta binaural beats, as defined above;
- an immersive video stream simulating or generating a virtual reality environment, as defined above;
- one soundtrack comprising sounds of nature, as defined above; and
- two sequences of alternating bilateral visual stimulations, as defined above.

In some embodiments, the digital therapeutic composition is a combination product, wherein at least some elements are provided individually for sequential administration or subjection to a subject.

This invention also relates to the digital therapeutic composition as described above, for use in therapy.

This invention also relates to the digital therapeutic composition as described above, for use in the treatment of pain in a subject in need thereof. It also relates to a method of treating pain in a subject in need thereof, comprising administering to the subject, or subjecting the subject to, the digital therapeutic composition as described above.

In some embodiment, pain is pelvic pain. In some embodiment, pain is chronic pelvic pain. In some embodiment, pain is endometriosis-related pain.

In some embodiments, the subject in need thereof is a woman.

In some embodiment, the subject in need thereof is a woman experiencing or affected with pelvic pain, preferably chronic pelvic pain, more preferably endometriosis-related pain.

In some embodiment, the subject in need thereof is a woman at risk of pelvic pain, preferably chronic pelvic pain, more preferably endometriosis-related pain.

In some embodiments, the virtual reality environment and the at least one oratorical soundtrack are to be administered to the subject in need thereof concomitantly and for the entire duration of the care. In some embodiments, the subject in need thereof is subjected to the virtual reality environment and the at least one oratorical soundtrack concomitantly and for the entire duration of the care.

In some embodiments, the at least one sequence of alpha binaural beats, the at least one sequence of theta binaural beats and the at least one sequence of alternating bilateral visual stimulations are to be administered to the subject in need thereof - or the subject in need thereof is subjected to the at least one sequence of alpha binaural beats, the at least one sequence of theta binaural beats and the at least one sequence of alternating bilateral visual stimulations - in the following order:
- a sequence of alpha binaural beats; then
- a first sequence of alternating bilateral visual stimulations; then
- a sequence of theta binaural beats; then
- optionally, a second sequence of alternating bilateral visual stimulations.

In some embodiments, the at least one sequence of alpha binaural beats, the at least one sequence of theta binaural beats and the at least one sequence of alternating bilateral visual stimulations are to be administered to the subject in need thereof - or the subject in need thereof is subjected to the at least one sequence of alpha binaural beats, the at least one sequence of theta binaural beats and the at least one sequence of alternating bilateral visual stimulations - in the following order:
- a sequence of alpha binaural beats; then
- a first sequence of alternating bilateral visual stimulations; then
- a sequence of theta binaural beats; then
- a second sequence of alternating bilateral visual stimulations.

In some embodiments, the at least one sequence of alpha binaural beats, the at least one sequence of theta binaural beats and the at least one sequence of alternating bilateral visual stimulations are to be administered to the subject in need thereof - or the subject in need thereof is subjected to the at least one sequence of alpha binaural beats, the at least one sequence of theta binaural beats and the at least one sequence of alternating bilateral visual stimulations - in the following order:
- a sequence of alpha binaural beats; then
- a single sequence of alternating bilateral visual stimulations; then
- a sequence of theta binaural beats.

In some embodiments, the sequence of alpha binaural beats is to be administered to the subject in need thereof - or the subject in need thereof is subjected to the sequence of alpha binaural beats - astride the first sequence of hypnosis induction and the second sequence of hypnosis injunction of the spoken hypnosis script of the at least one oratorical soundtrack, *i.e.*, the sequence of alpha binaural beats starts before the end of the first sequence of hypnosis induction and ends after the start of the second sequence of hypnosis injunction.

In some embodiments, the first sequence of alternating bilateral visual stimulation is to be administered to the subject in need thereof - or the subject in need thereof is subjected to the first sequence of alternating bilateral visual stimulations - during the sequence of hypnosis injunction of the spoken hypnosis script of the at least one oratorical soundtrack.

In some embodiments, the sequence of theta binaural beats is to be administered to the subject in need thereof - or the subject in need thereof is subjected to the sequence of theta binaural beats - during the sequence of hypnosis injunction of the spoken hypnosis script of the at least one oratorical soundtrack.

In some embodiments, the second sequence of alternating bilateral visual stimulations is to be administered to the subject in need thereof - or the subject in need thereof is subjected to the second sequence of alternating bilateral visual stimulations - during the sequence of hypnosis injunction of the spoken hypnosis script of the at least one oratorical soundtrack.

In some embodiments, the single sequence of alternating bilateral visual stimulations is to be administered to the subject in need thereof - or the subject in need thereof is subjected to the single sequence of alternating bilateral visual stimulations - during the sequence of hypnosis injunction of the spoken hypnosis script of the at least one oratorical soundtrack.

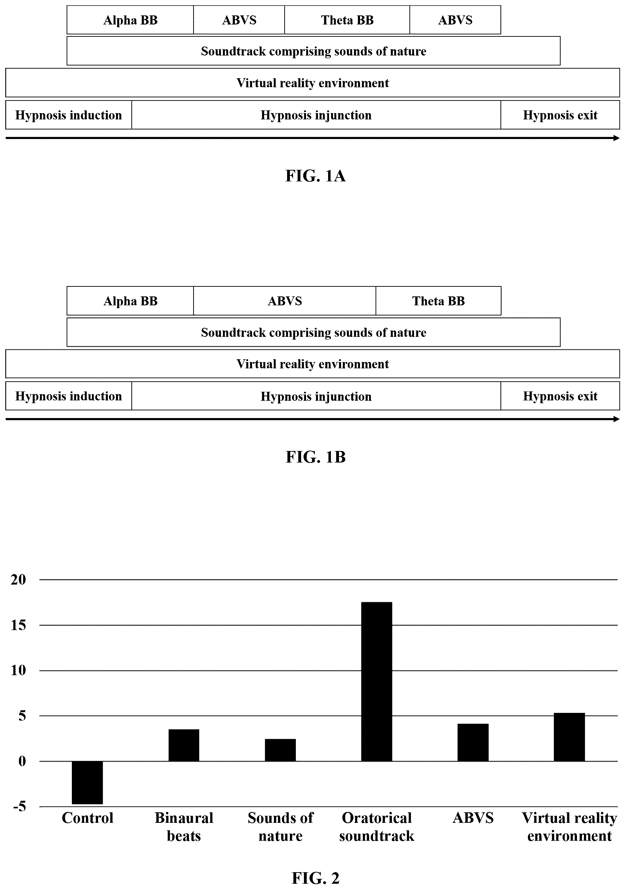

In some embodiments, the digital therapeutic composition is to be administered to the subject in need thereof - or the subject in need thereof is subjected to the digital therapeutic composition - according to the chronological sequence described in **Figure 1A or 1B****.**

This invention also relates to a non-therapeutic method of relaxation, comprising administering to a subject, or subjecting a subject to, the digital therapeutic composition as described above.

In some embodiments, the subject is a man or a woman.

In some embodiment, the subject is a man or a woman experiencing psychological stress, anxiety, and/or emotional strain.

In some embodiment, the subject is a man or a woman at risk of psychological stress, anxiety, and/or emotional strain.

In some embodiments, the virtual reality environment and the at least one oratorical soundtrack are to be administered to the subject concomitantly and for the entire duration of the care. In some embodiments, the subject is subjected to the virtual reality environment and the at least one oratorical soundtrack concomitantly and for the entire duration of the care.

In some embodiments, the at least one sequence of alpha binaural beats, the at least one sequence of theta binaural beats and the at least one sequence of alternating bilateral visual stimulations are to be administered to the subject- or the subject is subjected to the at least one sequence of alpha binaural beats, the at least one sequence of theta binaural beats and the at least one sequence of alternating bilateral visual stimulations - in the following order:
- a sequence of alpha binaural beats; then
- a first sequence of alternating bilateral visual stimulations; then
- a sequence of theta binaural beats; then
- optionally, a second sequence of alternating bilateral visual stimulations.

In some embodiments, the at least one sequence of alpha binaural beats, the at least one sequence of theta binaural beats and the at least one sequence of alternating bilateral visual stimulations are to be administered to the subject- or the subject is subjected to the at least one sequence of alpha binaural beats, the at least one sequence of theta binaural beats and the at least one sequence of alternating bilateral visual stimulations - in the following order:
- a sequence of alpha binaural beats; then
- a first sequence of alternating bilateral visual stimulations; then
- a sequence of theta binaural beats; then
- a second sequence of alternating bilateral visual stimulations.

In some embodiments, the at least one sequence of alpha binaural beats, the at least one sequence of theta binaural beats and the at least one sequence of alternating bilateral visual stimulations are to be administered to the subject- or the subject is subjected to the at least one sequence of alpha binaural beats, the at least one sequence of theta binaural beats and the at least one sequence of alternating bilateral visual stimulations - in the following order:
- a sequence of alpha binaural beats; then
- a single sequence of alternating bilateral visual stimulations; then
- a sequence of theta binaural beats.

In some embodiments, the sequence of alpha binaural beats is to be administered to the subject - or the subject is subjected to the sequence of alpha binaural beats - astride the first sequence of hypnosis induction and the second sequence of hypnosis injunction of the spoken hypnosis script of the at least one oratorical soundtrack, *i.e.*, the sequence of alpha binaural beats starts before the end of the first sequence of hypnosis induction and ends after the start of the second sequence of hypnosis injunction.

In some embodiments, the first sequence of alternating bilateral visual stimulation is to be administered to the subject - or the subject is subjected to the first sequence of alternating bilateral visual stimulations - during the sequence of hypnosis injunction of the spoken hypnosis script of the at least one oratorical soundtrack.

In some embodiments, the sequence of theta binaural beats is to be administered to the subject - or the subject is subjected to the sequence of theta binaural beats - during the sequence of hypnosis injunction of the spoken hypnosis script of the at least one oratorical soundtrack.

In some embodiments, the second sequence of alternating bilateral visual stimulations is to be administered to the subject - or the subject is subjected to the second sequence of alternating bilateral visual stimulations - during the sequence of hypnosis injunction of the spoken hypnosis script of the at least one oratorical soundtrack.

In some embodiments, the single sequence of alternating bilateral visual stimulations is to be administered to the subject - or the subject is subjected to the single sequence of alternating bilateral visual stimulations - during the sequence of hypnosis injunction of the spoken hypnosis script of the at least one oratorical soundtrack.

In some embodiments, the digital therapeutic composition is to be administered to the subject - or the subject is subjected to the digital therapeutic composition - according to the chronological sequence described in **Figure 1A or 1B**.

The invention also relates to is a system **1** for the implementation of a therapeutic method for pain relief. It also relates to a system **1** for the implementation of a non-therapeutic method of relaxation.

The system comprises a display and stereo headphones, configured to administer to a subject the digital therapeutic composition as described above.

In some embodiments, the system **1** comprises a virtual reality head-mounted device having at least a head-mounted display **2** and stereo headphones **3**; at least a storage medium **4**; and a controlling unit **5** of the virtual reality head-mounted device.

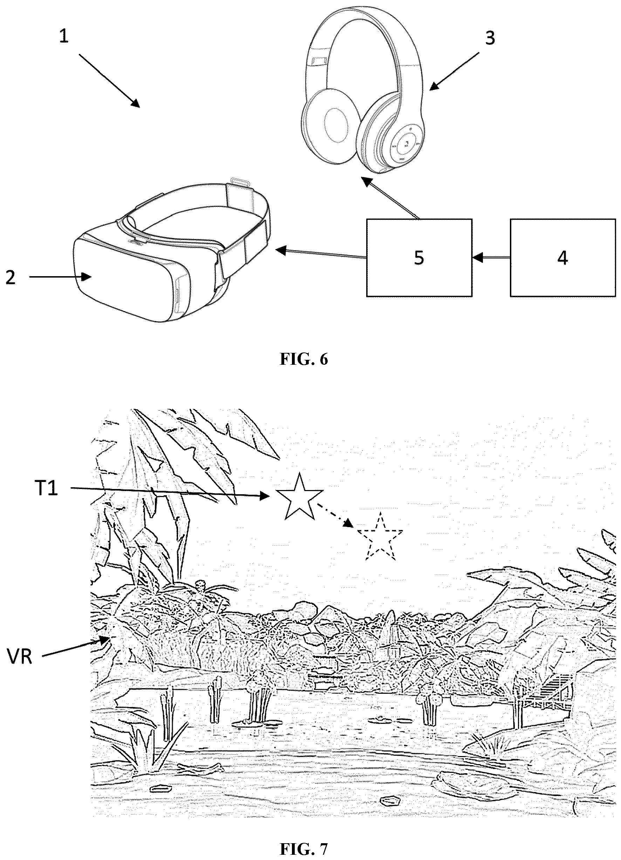

As shown in **Figure 6**, the system **1** comprises a storage medium **4**, which might be a memory of the head-mounted display **2**, a memory of a smartphone in connection, by wire or wireless, with the head-mounted display **2** or a memory of a distant server with which the head-mounted display **2** exchanges data. The system **1** also comprises a controlling unit **5** of the head-mounted display **2** and the stereo headphones **3**, said controlling unit **5** being able to stream immersive video files, stored in the storage medium **4**, through the head-mounted display **2** and soundtracks, stored in the storage medium **4**, through the stereo headphones **3.**

A plurality of digital components is stored in the storage medium **4**, forming altogether the digital therapeutic composition described above. These components are depicted in **Figure 1A and 1B**.

A first digital component comprises an immersive video stream simulating or generating a virtual reality environment, as described above. In a non-limitative embodiment, the virtual reality environment may represent a lagoon surrounded by a forest. In a non-limitative embodiment, the immersive video stream can further comprise one or several subsets of images representing a graphical trigger element. **Figure 7** shows an exemplary representation of such virtual reality environment with a graphical trigger element (here, a shooting star sailing through the sky).

A second digital component comprises at least one sequence of alpha binaural beats and at least one sequence of theta binaural beats, as described above.

A third digital component comprises at least one oratorical soundtrack, which comprises or consists of a spoken hypnosis script, as described above.

A fourth digital component comprises at least one sequence of alternating bilateral stimulations, as described above. In some embodiments, the alternating bilateral stimulations are alternating bilateral visual stimulations comprising or consisting of a visual stimulus moving in a rhythmic left-right pattern.

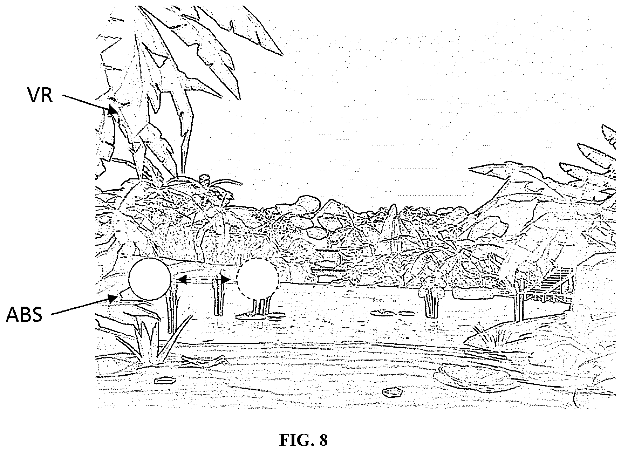

In a non-limitative embodiment, the visual stimulus may be a sphere that appears or otherwise forms in the virtual reality environment. **Figure 8** shows an exemplary representation of such virtual reality environment with a sequence of alternating visual bilateral stimulations.

A fifth digital component comprises at least one soundtrack comprising sounds of nature, as described above.

In some embodiments, at least a first, a second and a third digital components are stored on the storage medium **4.**

In some embodiments:
- the first digital component comprises an immersive video stream simulating or generating a virtual reality environment;
- the second digital component comprises at least one sequence of alpha binaural beats and at least one sequence of theta binaural beats;
- the third digital component comprises at least one oratorical soundtrack, which comprises or consists of a spoken hypnosis script.

In some embodiment, a fourth and a fifth digital component, as described above, are further stored on the storage medium **4.**

Following the reception of a trigger instruction to the system **1**, the controlling unit **5** streams the digital components through the virtual reality head-mounted device comprising the head-mounted display **2** and the stereo headphones **3.**

Embodiment **E1**: a therapeutic system for the implementation of a therapeutic method for pain relief, comprising a virtual reality head-mounted device having at least a head-mounted display and stereo headphones; said system comprising at least a storage medium and a controlling unit of the virtual reality head mounted device, wherein at least a first, a second and a third therapeutic components are stored on the storage medium; wherein:
- said first therapeutic component comprises an immersive video stream intended to generate a virtual reality environment in which a patient has to be immersed;
- said second therapeutic component comprises a first soundtrack comprising a first and a second successive sequences of binaural beats, the beats frequencies differences being different from one binaural beats sequence to the other;
- said third therapeutic component comprises a second soundtrack comprising a spoken hypnosis script;
and wherein, following the reception of a trigger instruction of the therapeutic method, said controlling unit is configured to simultaneously stream the first, the second and the third therapeutic components through the virtual reality head mounted device.

Embodiment **E2**, according to **E1**: the first sequence of binaural beats comprises an alpha binaural beats sequence comprising two dichotic pure-tone sine waves, with a frequency difference ranging from about 9 to about 14 Hz; and wherein the second sequence of binaural beats comprises a theta binaural beats sequence following said alpha binaural beats sequence and comprising two dichotic pure-tone sine waves, with a frequency difference ranging from about 4 to about 8 Hz.

Embodiment **E3**, according to **E1** or **E2**: the second soundtrack comprises:
- a first hypnosis induction block of predefined sentences spoken by a human voice;
- a second hypnosis injunction block of predefined sentences spoken by a human voice; and
- a third hypnosis exit block of predefined sentences spoken by a human voice.

Embodiment **E4**, according to any one of **E1** to **E3**: each block is spoken by a same woman characterized by a voice frequency ranging from about 150 to about 300 Hz.

Embodiment **E5**, according to **E3** or **E4**: the voice rhythm and/or the voice pitch and/or the voice loudness and/or the voice harmonic to noise ratio decreases along the second hypnosis injunction block.

Embodiment **E6**, according to any one of **E3** to **E5**: the first sequence of binaural beats is to be streamed astride the streaming of the first hypnosis induction block and the second hypnosis injunction block; and the second sequence of binaural beats is to be streamed during the streaming of the second hypnosis injunction block.

Embodiment **E7**, according to any one of **E1** to **E6**: the immersive video stream comprises a sequence of images representing said virtual reality environment and in which at least a subset of images represents the appearance and the disappearance of a graphical trigger element in said virtual reality environment.

Embodiment **E8**, according to **E7**: the first or the second sequence of binaural beats is to be streamed simultaneously with the streaming of said subset of images.

Embodiment **E9**, according to any one of **E7** or **E8** in combination with any one of **E3** to **E6**: the immersive video stream comprises a first sequence of images representing said virtual reality environment with a first dominant color and a second sequence of images, immediately following the first sequence of images and representing said virtual reality environment with a second dominant color distinct from the first dominant color, and wherein the first sequence of binaural beats is to streamed with or after the beginning of the streaming the second sequence of image.

Embodiment **E10**, according to any one of **E1** to **E9**: at least a fourth therapeutic components is stored on the storage medium; wherein said fourth therapeutic component comprises an alternative bilateral stimulation video stream representing an object having an alternating motion pattern, and wherein, following the reception of a trigger instruction of the therapeutic method, said controlling unit is configured to simultaneously stream the first, the second, the third and the fourth therapeutic components through the virtual reality head mounted device.

Embodiment **E11**, according to **E10**: said alternating motion pattern is a rhythmic left-right pattern with a frequency ranging from about 0.2 Hz to about 2 Hz.

Embodiment **E12**, according to **E10** or **E11**: the alternative bilateral stimulation video stream comprises at least a first and a second sequence of images each representing an alternative bilateral stimulation, wherein said first sequence of images is to be streamed between the end of the streaming of the first sequence of binaural beats and the beginning of the streaming of the second sequence of binaural beats; and wherein said second sequence of images is to be streamed after end of the streaming of the second sequences of binaural beats.

Embodiment **E13**, according to **E12** in combination with any one of **E3** to **E6**: the third hypnosis exit bloc is to be streamed with or after the end of the streaming of the second sequence of images.

Embodiment **E14**, according to any one of **E1** to **E13**: at least a fifth therapeutic components is stored on the storage medium; wherein said fifth therapeutic component comprises a third soundtrack comprising sounds of nature; and wherein, following the reception of a trigger instruction of the therapeutic method, said controlling unit is configured to simultaneously stream the first, the second, the third, the fourth and the fifth therapeutic components through the virtual reality head mounted device.

Embodiment **E15**, according to **E14**: during the streaming of the therapeutic components, said controlling unit is configured to modulate the sound volume of the third soundtrack for each left and right speakers of the stereo headphones according to the spatial orientation of the head-mounted display.

Embodiment **E16**: virtual reality method intended to be implemented in a therapeutic system according to any one of **E1** to **E15**, said method comprising a step of simultaneously streaming, through the virtual reality head mounted device of the therapeutic system:
- said immersive video stream stored on the storage medium to generate a virtual reality environment in which the patient is immersed;
- said first soundtrack stored on the storage medium comprising a first and a second successive sequences of binaural beats;
- said second soundtrack stored on the storage medium comprising a spoken hypnosis script.

Embodiment **E17**: computer program product recorded on a computer storage device, the computer program product including instructions that when executed by a processor cause the processor to execute the steps of a method according to **E16**.

Embodiment **E18**: computer-readable storage medium including portions of code of a computer program intended to be executed by the controlling unit of a therapeutic system according to according to any one of **E1** to **E15** so as to implement the method according to **E16**.

Embodiment **E19** according to **E18**: the computer-readable storage medium further contains at least the first, the second and the third therapeutic components.

### BRIEF DESCRIPTION OF THE DRAWINGS

**Figures 1A-B** are exemplary timelines of the digital therapeutic composition described herein. Alpha BB: sequence of alpha binaural beats; Theta BB: sequence of theta binaural beats; ABVS: sequence of alternating bilateral visual stimulations. **Figure 1A** shows an exemplary timeline comprising two sequences of alternating bilateral visual stimulations; **Figure 1B** shows an exemplary timeline comprising a single sequence of alternating bilateral visual stimulations.
**Figure 2** is a graph showing the mean difference of pain felt by 8 participants between the end of a 60-second pain administration and the end of treatment with either of binaural beats, sounds of nature, an oratorical soundtrack comprising a hypnosis script, alternating bilateral visual stimulations or a virtual reality environment.
**Figure 3** is a mixed model comparison of pain perception before (T0) treatment (Endocare or control), with the clustered post-treatment pain evaluations at T15, T30, T45, T60 and T240. ^{∗}: *p* < 0.05.
**Figure 4** is a mixed model comparison of pain perception for each of the five post-treatment evaluations (T15, T30, T45, T60 and T240) to before treatment (T0). *^{∗}*: *p* < 0.05 for each post-test time for individual groups (Endocare and control); ⁺: *p* < 0.05 for each interaction group (group × time).
**Figure 5** is a graph showing the perceived pain relief rate at the end of each measurement after treatment. +: *p* < 0.05.
**Figure 6** is a simplified representation of a system 1 for implementing a therapeutic method for pain relief or a non-therapeutic method of relaxation.
**Figure 7** is an instant 2-dimension view of a virtual reality environment (VR) and graphical trigger element (T1).
**Figure 8** is an instant 2-dimension view of a virtual reality environment (VR) and a sequence of alternating bilateral visual stimulations (ABS).

### EXAMPLES

The present invention is further illustrated by the following examples.

### Example 1

The aim of this experiment was to define the therapeutic potential of each element of the digital therapeutic composition taken individually.

### Materials and Methods

Eight healthy participants (5 women, 3 men, from 24 to 36 years old) were exposed to a conditioned pain modulation paradigm by putting their hands in cold water for 60 seconds. The participants were then subjected to binaural beats, sounds of nature, an oratorical soundtrack comprising a hypnosis script, alternating bilateral visual stimulations or a virtual reality environment.

Pain was rated at the end of the 60-second immersion in water, and after treatment (subjection to each of the digital therapeutic composition elements taken individually).

### Results

Results are shown in **Figure 2**, representing the mean difference of pain felt by the participants between the end of the 60-second immersion in water and the end of treatment.

As seen in this figure, the oratorical soundtrack comprising a hypnosis script provided by far the best therapeutic efficacy for alleviating pain. Binaural beats, sounds of nature, alternating bilateral visual stimulations and virtual reality environment also showed a therapeutic efficacy for alleviating pain, although to a lesser extent.

Conversely, when the participants were not subj ected to any treatment after their 60-second immersion in water (control), pain further amplified.

### Example 2

### Material and methods

This was a randomized, controlled, comparative, open-label, two-parallel-group, interventional study comparing the effect of the digital therapeutic composition described herein (and hereafter named "**Endocare**") and a digital control on endometriosis-related pain after a single use. This randomized controlled trial was conducted between December 2020 and May 2021 in Clinic Tivoli-Ducos in Bordeaux, France.

The study was conducted in compliance with good clinical practice (GCP) guidelines, the principles of the "Declaration of Helsinki", and with the French laws and regulations. The study was reviewed and approved by the ethic committee. The subject completed and signed an informed consent form (ICF) before her inclusion in the study and before any study-related procedure started.

### Population

The selection criteria were women over 18 with a diagnosis of endometriosis willing to participate in the study and having signed the informed consent form.

The screened participants meeting the selection criteria had to be suffering from a moderate-to-severe endometriosis-related pain ≥ 4 on numerical rating scale (NRS 0 to 10) at the time of inclusion, a criterion shared among various studies.

The non-selection criteria were pregnant or breastfeeding women; women having consumed painkillers within 8 hours prior to inclusion; women participating in an interventional study or having participated in an interventional study within the 30 days before enrolment; women being an employee of the investigator or study site, with direct involvement in the proposed study or other studies under the direction of that investigator or study site, as well as family members of the employees or the investigator; women with contraindication to Endocare or digital control (subjects with severe visual, hearing and/or cognitive impairments, subjects with color blindness, subjects who are photo-sensitive or suffering from epilepsy or motion sickness).

45 subjects were included and randomized in this trial, and 44 of them completed the study.

Pain medications were stopped at least 8 hours prior to participation. After treatment (Endocare or control), if pain persisted and was not relieved by treatment, the patient could take their pain management rescue treatment. If the patient used painkiller during the follow-up period, the study stopped for the patient at the time the rescue medication was taken.

### Treatment and control

The Endocare treatment developed by the Inventors was displayed through a VR headset (Oculus Quest) associated with a high-quality headphone (APK K-240-MKII). Endocare treatment is a stand-alone software medical device, which is composed of an application stored in a VR headset, intended to mitigate the pain for subjects prone to endometriosis. Endocare offers a 20-minute treatment consisting of a combination of auditory and visual therapeutic procedures integrated in a 3-D VR environment.

The digital control was developed specifically for this study. It was displayed through a tablet (Samsung Galaxy Tab A) associated with a high-quality headphone similar to those used in the Endocare group (APK K-240-MKII). The digital comparator was a 20-minute control with the same composition as the Endocare treatment (same context, same environment, same duration) but without visual and auditory immersion. A soundtrack composed of nature sounds related to the projected image.

### Procedures

Women suffering from endometriosis pain were recruited from the "Centre Endométriose, Clinique Tivoli-Ducos" in Bordeaux, France, specialized in endometriosis. All patients were diagnosed with endometriosis by specialized gynecologists. The recruitment was done at the moment the patients came for their medical examination and were informed about the project. If the patients were interested in participating in the study, the research team offered them a new appointment at the Clinique to receive the treatment.

When they returned to the clinic on the day of the inclusion visit, their pain level needed to be at least 4 on a 0-to-10 numeric rating scale to be able to participate to the study. Subjects reporting a pain < 4 on this scale were considered as screen failures and withdrawn from the study. This pain intensity evaluation represents the pre-treatment evaluation just before the use of Endocare or control and was considered as baseline (T0).

If the patient accepted to participate and the inclusion criteria were completed, the patient was randomized to one of the two groups, Endocare or control. The treatment (either Endocare or control) consisted in a single use on the day of the visit.

The follow-up duration lasted for 240 minutes (T240) following the treatment with Endocare or control.

Once the treatment was completed, the patient was asked to rate on a paper questionnaire the pain relief on a 5-points categorical scale and pain intensity based on the 11-points NRS at 15 minutes (T15), 30 minutes (T30), 45 minutes (T45) and 60 minutes (T60) after administration of the allocated study treatment. During the first hour, the subject remained under the direct supervision of the study site staff. After the first hour and if cleared by the study site staff, the subject was free to leave study site and go home to complete T240 assessments. Adverse events, if any, were collected during the entire trial.

At home, the patient was asked to rate on a paper questionnaire the pain relief on a 5-points categorical scale and pain intensity based on the 11-points NRS at T240, after treatment. At T240, or at last time point if the subject dropped out the study or started a rescue medication before the end of the follow-up, the subject was asked to rate her satisfaction about Endocare or the control after a single use on a dedicated questionnaire.

### Measurements

The patient was asked to rate the pain intensity on 11-points numeric rating scale (0 = no pain; 10 = unbearable pain) at T0, T15, T30, T45, T60, and T240.

If rescue medication was needed, the patient was asked to rate her pain intensity just prior to the intake of the rescue medication. Study follow-up was terminated upon the intake of any rescue medication.

The patient was asked to rate her pain relief on a 5-points categorical scale (0 = no relief, 1 = slight relief, 2 = moderate relief, 3 = important relief, 4 = complete relief) at T15, T30, T45, T60 and T240 after administration of the allocated study treatment.

If the patient took rescue medication, she was asked to rate the pain relief just prior to the intake of the rescue medication. Study follow-up was terminated upon the intake of rescue medication.

### Statistics

A mixed-model framework was used for statistical analysis (Statistical Package for the Social Sciences, 2020). The selected covariance matrix of the repeated measurements that better fitted the data based on the Akaike Information Criterion (AIC) was the first-order ante-dependence. There were three explanatory fixed factors: group (Endocare, Control), time (T0, T15, T30, T45, T60, T240) and group × time and a random effect on the intercept of each participant. In the first analysis, time (fixed effect) was parametrized as baseline (T0) vs all other times (T15 to T240). The analysis consisted in comparing T0 with the five post-treatment data clustered for both groups and look at the interaction term (group × time). A contrast analysis was also performed to verify the difference between baseline (T0) and post-treatment times (clustered) in the two groups separated. In the second analysis, time was parametrized as is (T0, T15, T30, T45, T60 and T240). A contrast analysis was also performed to test the difference between T0 and all other times (separated) in both groups separated. Bonferroni corrections were applied for multiple comparisons.

For pain relief, data were analyzed using R software (stats package). As data did not satisfy to normality (assessed with Shapiro-Wilk normality test), Wilcoxon unilateral unpaired tests corrected for multiple comparisons with FDR were used.

### Results

### Study participants

Of the 45 women suffering from endometriosis that participated in the study, one didn't return the evaluation at T240. The results for the time T240 from 5 patients who took a rescue medication before that measure (4 from the control group and 1 from the treatment group), were excluded from the analysis. The patients in the two groups were comparable for age, height and weight.

All the participants were recruited from a specialized clinic in endometriosis and were majorly suffering from severe symptoms. 90 % were suffering from chronic pelvic pain not related to menses, and the majority were suffering from dysmenorrhea, dysuria and dyspareunia. 77 % percent were suffering from deep infiltrating endometriosis, and 22 % had adenomyosis. Close to 70 % of the patients had surgery for endometriosis.

The majority of the patients were taking different classes of medications for their endometriosis condition, including hormones, analgesics and antidepressant. Since the study was based on an add-on protocol, the subjects were authorized to continue all their medications, with the exception of the short-acting pain drugs before the beginning of the testing. The patients kept these analgesics as rescue medication.

### Pain perception

### Difference in pain perception before (T0) and the clustered five post-treatments

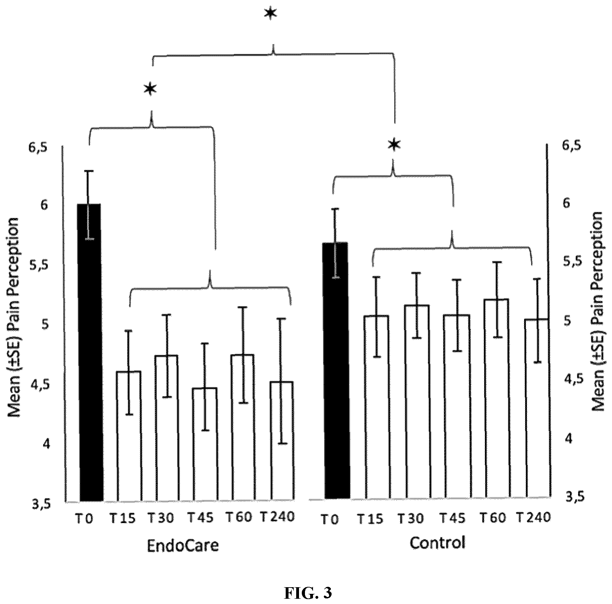

We compared the difference between the baseline pain (T0) and the clustered five post-treatment measurements covering 15 minutes to 4 hours after the treatment (T15, T30, T45, T60, T240). Both the treatment (Endocare) and the control groups significantly reduced the pain perception (Endocare: mean reduction = 1.38, *p* = 0.001; Control: mean reduction = 0.58, *p* = 0.008). The group (Endocare, Control) × times (T0, post-all) was also significantly different (F = 7.343, *p* = 0.010), Endocare being significantly more efficient than the control (**Figure 3**).

### Comparison of the pain perception between T0 and each post-treatment (T15, T30, T45, T60, T240)

In the same mixed model, we looked for the effect on the differences between T0 and each post-treatment measures, from T15 to T240, and the Group × Time interaction. The results were adjusted for multiple comparisons with Bonferroni corrections. The five post-treatment measures were significantly different from T0 for the treatment group. For the control group, except for a tendency for T15 (*p* = 0.068), none of the post-treatment group were significantly reducing pain perception (*all p* > 0.05).

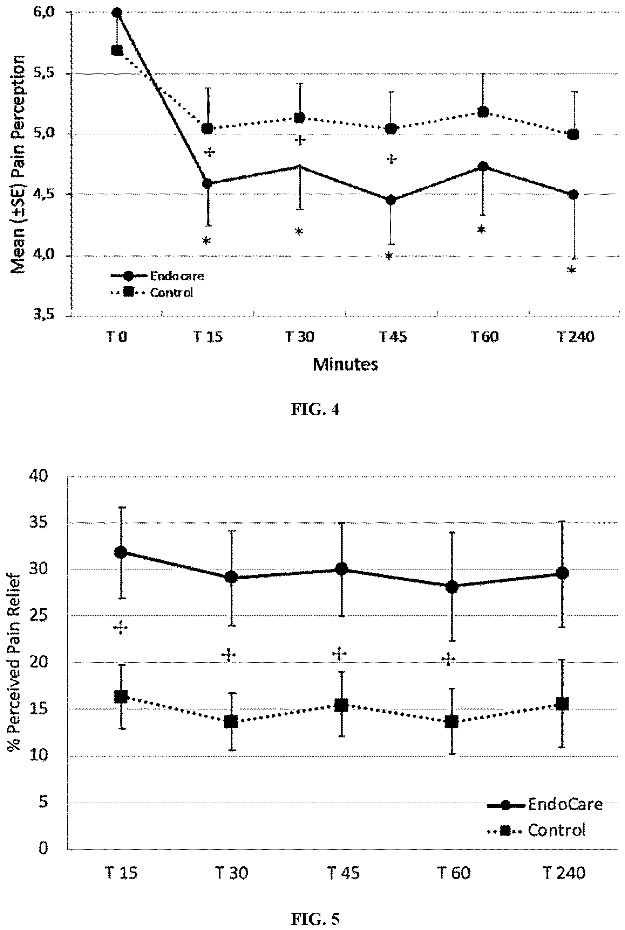

For the interaction Group × Time, T15 to T45 were significantly different between the two groups (all *p* < 0.001). Endocare was significantly more efficient than the control group from 15 minutes to 45 minutes post-treatment (**Figure 4**).

### Covariance for adenomyosis and surgery

Since 31 patients had surgery related to endometriosis (Endocare: 14; Control: 17), and 11 suffered from adenomyosis (Endocare: 6; Control: 5), we introduced two covariates (adenomyosis and surgery) in the mixed model. Neither adenomyosis nor surgery changed the results (adenomyosis: *p* = 0.924; surgery: *p* = 0.982). These results confirmed that the effect of the treatment was independent of the potential complication related to adenomyosis and surgery.

### Pain Relief

At each post-treatment measurement, the patients were asked to measure their perceived pain relief on a 0-to-4 numerical scale from no relief to total relief. The mean pain relief reported for Endocare was 28 % (range: 26 to 30 %) *versus* 15 % for the control (range: 14 to 16 %). Non parametric Wilcoxon unilateral unpaired tests corrected for multiple comparisons with FDR were performed. All post-treatment times presented a significantly higher pain relief score for Endocare compared to the control group (p < 0.05) (**Figure 5**).

### Maximum reduction in pain as assessed by NRS - Intent-to-treat analysis

We compared the maximum reduction of pain in both groups. The mean maximum effect was 42 % (95 % CI: 30.82; 53.18) for Endocare and 22 % (95 % CI: 15.38; 28.53) for the control group. The maximum effect was significantly higher for the treatment group (p = 0.004).

### Adverse Events

Seven subjects (15 %) reported mild to moderate adverse events, four were evaluated as probably unrelated (8 %) and three possibly related (6 %) to Endocare treatment. The three possibly related events were in the treatment group and were described as a mild headache and/or nausea related to motion sickness.

### Conclusion

In this randomized controlled trial, placebo-control study, we aimed at measuring the immediate and persisting effect of a DTx (Endocare) on pain in women suffering from pelvic pain related with endometriosis.

We hypothesized that Endocare would be superior to the control at reducing clinical pain perception. Both Endocare and the control reduced the overall pain perception when comparing the pain at baseline (T0) with the combined five post-treatment measurements from 15 minutes (T15) to 4 hours (T240).

However, Endocare was significantly superior to the control. The mean effect was 1.38 for Endocare and 0.58 for the control. The percent of the perceived pain reduction ranged between 26 % to 30 % for Endocare, and from 14 % to 16 % for the control. These results were partially explained by the cohort of our study, that comprised women with severe endometriosis.

To assess whether the beneficial effect was lessened for population with complex pathologies related to endometriosis, we covariated for surgery and adenomyosis, two conditions with potentially more pain and less responses to the treatment. We found that the results were equivalent, suggesting a similar effect in all subpopulations, including the more complex pathophysiologies.

Previous studies had shown that a virtual reality environment was an effective way to reduce acute pain intensity, especially pain experienced during medical procedures, burn victims, or by women during childbirth. Some studies have also looked at the effects of VR on chronic pain, and reported that VR was effective to reduce, *e.g.*, chronic low back pain throughout the duration of the treatment.

In our study, to potentialize VR effects, we chose additional auditory and visual stimulations, to offer a DTx treatment that could act on different components of pain: alpha and theta binaural beats, sounds of nature, alternating bilateral visual stimulations, and hypnosis.

The combination of these stimulations coupled with the VR experience led to the decrease in pain observed in the Endocare group immediately after and up to 4 hours post-treatment.

To our knowledge, our study is the first to demonstrate an effect on pain reduction in women diagnosed and suffering from moderate-to-severe endometriosis-related pelvic pain using a non-pharmacological treatment combining VR with visual and auditory stimuli.

## Claims

1. A digital therapeutic composition, comprising:
- at least one oratorical soundtrack;
- at least one sequence of alpha binaural beats; and
- at least one sequence of theta binaural beats;
and further optionally comprising:
- an immersive video stream simulating or generating a virtual reality environment;
- at least one soundtrack comprising sounds of nature; and/or
- at least one sequence of alternating bilateral stimulations.

2. The digital therapeutic composition according to claim **1**, wherein the oratorical soundtrack comprises a hypnosis script, preferably said hypnosis script is spoken by a woman **characterized by** a voice frequency ranging from about 150 to about 300 Hz.

3. The digital therapeutic composition according to claim **1** or **2**, wherein the oratorical soundtrack comprises:
a) a first sequence of hypnosis induction;
b) a second sequence of hypnosis injunction; and
c) a third sequence of hypnosis exit.

4. The digital therapeutic composition according to any one of claims **1** to **3**, wherein the at least one sequence of alpha binaural beats and the at least one sequence of theta binaural beats each independently comprise two dichotic soundtracks, wherein each dichotic soundtrack comprises a pure-tone sine wave with a frequency ranging from about 450 to about 500 Hz.

5. The digital therapeutic composition according to any one of claims **1** to **4**, wherein the virtual reality environment comprises at least one sequence of images in natural environment or in a fantasy environment.

6. The digital therapeutic composition according to any one of claims **1** to **5**, wherein the sounds of nature are linked or related to at least one sequence of images of the virtual reality environment.

7. The digital therapeutic composition according to any one of claims **1** to **6**, wherein the at least one sequence of alternating bilateral stimulations comprises a visual stimulus.

8. The digital therapeutic composition according to claim **7**, wherein the visual stimulus moves in a rhythmic left-right pattern with a frequency ranging from about 0.2 Hz to about 2 Hz, preferably with a frequency of about 0.8 Hz.

9. The digital therapeutic composition according to any one of claims **1** to **8**, said composition comprising:
- an oratorical soundtrack,
- one sequence of alpha binaural beats,
- one sequence of theta binaural beats,
- an immersive video stream simulating a virtual reality environment,
- a soundtrack comprising sounds of nature, and
- at least one sequence of alternating bilateral visual stimulations.

10. The digital therapeutic composition according to any one of claims **1** to **9**, for use in therapy.

11. The digital therapeutic composition according to any one of claims **1** to **9**, for use in the treatment of pain in a subject in need thereof.

12. The digital therapeutic composition for use according to claim **11**, wherein pain is pelvic pain, preferably chronic pelvic pain, more preferably endometriosis-related pain.

13. The digital therapeutic composition for use according to any one of claims **10** to **12**, wherein the virtual reality environment and the at least one oratorical soundtrack are to be administered to the subject in need thereof concomitantly and for the entire duration of the care.

14. The digital therapeutic composition for use according to any one of claims **10** to **13**, wherein the at least one sequence of alpha binaural beats, the at least one sequence of theta binaural beats and the at least one sequence of alternating bilateral stimulations are to be administered to the subject in need thereof in the following order:
- the at least one sequence of alpha binaural beats is to be administered astride a first sequence of hypnosis induction and a second sequence of hypnosis injunction; then
- a first sequence of alternating bilateral stimulations is to be administered during the second sequence of hypnosis injunction; then
- the at least one sequence of theta binaural beats is to be administered during the second sequence of hypnosis injunction; then
- optionally, a second sequence of alternating bilateral stimulations is to be administered during the second sequence of hypnosis injunction.

15. A non-therapeutic method of relaxation, comprising subjecting a subject to the digital therapeutic composition according to any one of claims **1** to **9**.
